# EUROPEAN PATENT SPECIFICATION

(11) **EP 1 529 841 B1**
(45) Date of publication and mention of the grant of the patent: **19.09.2007**
(21) Application number: 04026296.6
(22) Date of filing: 05.11.2004
(51) Int. Cl.: C12N 15/10, C12Q 1/68

(54) **RNA extraction method, RNA extraction reagent, and method for analyzing biological materials**
Methode und Reagentien zur Extraktion von RNA und Methode zur Analyse von biologischem Material
Procédé et kit d'extraction d'ARN et méthode pour l'analyse de matériaux biologiques

(30) Priority: 07.11.2003 JP 2003378516
(43) Date of publication of application: 11.05.2005
(73) Proprietor: Hitachi High-Technologies Corporation, Tokyo 105-8717 (JP)
(72) Inventor: Yamashita, Yoshihiro, c/o Hitachi High-Techn. Corp, Hitachinaka-shi, Ibaraki 312-8504 (JP); Sakurai, Toshinari,c/o Hitachi High-Techn. Corp., Hitachinaka-shi, Ibaraki 312-8504 (JP); Kuno, Norihito, Hitachi, Ltd., Chiyoda-ku, Tokyo 100-8220 (JP); Uchida, Kenko, c/o Hitachi, Ltd., Chiyoda-ku, Tokyo 100-8220 (JP); Yokobayashi, Toshiaki, c/o Hitachi High-Tech. Corp, Hitachinaka-shi, Ibaraki 312-8504 (JP)
(74) Representative: Strehl Schübel-Hopf & Partner

(56) References cited:
- WO-A-95/21849
- DE-A- 19 912 799
- US-A- 5 155 018
- US-A1- 2002 192 667
- DATABASE WPI Section Ch, Week 199810 Derwent Publications Ltd., London, GB; Class B04, AN 1998-104112 XP002312865 & JP 09 327291 A (TOYOBO KK) 22 December 1997 (1997-12-22)

## Description

### FIELD OF THE INVENTION

The present invention relates to RNA extraction from biological materials containing RNA and a method for analyzing biological materials containing RNA.

### BACKGROUND OF THE INVENTION

While DNA is the substance that carries the total genetic information of organisms, RNA is the substance that plays an important role in protein biosynthesis *in vivo* on the basis of genetic information. Lately, gene sequence information of a number of organisms has been clarified by analysis of DNA. As a consequence of this, the elucidation of gene functions by RNA analysis is of increasing importance, and the procedure to isolate RNA from biological materials has become essential. RNA analysis methods include principally reverse transcriptase-polymerase chain reaction (RT-PCR), Northern blotting, and the like.

To obtain satisfactory results in these analysis methods, the use of RNA with high purity is required. Particularly in the RT-PCR, RNA analysis becomes difficult when DNA is present with RNA. Accordingly, it is desired that RNA is isolated in high purity not contaminated with DNA, proteins, lipids, carbohydrates, and the like that are present in cells.

A commonly used RNA extraction method is AGPC method. The AGPC method includes the following steps: (1) Dissolve a biological material in a solution of guanidine thiocyanate, then add an acid buffer solution, phenol solution, and chloroform solution successively, and mix. (2) Separate the mixed solution by centrifugation to an aqueous phase containing RNA and an intermediate phase, between an organic phase and the aqueous phase, containing denatured proteins and insolubilized DNA. (3) Add ethanol or isopropanol to the aqueous solution containing RNA. (4) Precipitate selectively the insolubilized RNA by centrifugation.

Extraction methods of nucleic acids that neither use toxic chemicals such as phenol and chloroform nor require a relatively long-time consuming procedure such as ethanol precipitation or isopropanol precipitation include a method in which nucleic acids are recovered from agarose gel by taking advantage of the ability of nucleic acids to bind to silica in the presence of a chaotropic agent and another method in which nucleic acids are extracted from biological materials using a chaotropic agent and silica particles. However, these methods have no selectivity between RNA and DNA, and the nucleic acid extracts are present in a mixture of RNA and DNA. Therefore, a procedure to remove DNA contained in the nucleic acid extracts is sometimes required for RNA analysis. The removal of DNA is mainly carried out by DNase treatment, followed by a procedure to remove the enzyme as appropriate. In general, approximately one hour of treatment time with DNase is necessary for the procedure to remove DNA. Moreover, the removal of the enzyme requires complicated procedures such as phenol/chloroform extraction and ethanol precipitation, thus resulting in a loss of RNA.

There exists a selective extraction method of RNA by taking advantage of the ability of RNA to bind to silica in the presence of a chaotropic agent and an organic solvent (JP-A No. 187897/2002). In this method, the difference between the binding abilities of DNA and RNA to silica is controlled by adding ethanol, isopropanol, or the like to a chaotropic agent, thereby allowing RNA to bind to silica selectively. The selectivity of this method toward RNA is, however, insufficient, and a procedure to remove DNA contaminated in the nucleic acid extracts is needed.

US 2002/0192667 A1 discloses a method for isolating and purifying nucleic acids from biological samples comprising providing a mixed solution containing nucleic acids, a chaotropic agent and an organic solvent, adsorbing the nucleic acids on an adsorption support, and desorbing the nucleic acids from the support after washing.

DE 19912799 A1 discloses a method of selectively eluting DNA and RNA from a solid phase to which DNA and RNA are adsorbed.

JP 09-327291 A discloses a RNA extraction technique for causing RNA from a biological sample in which RNA and DNA coexist to selectively bind to a solid phase using phenol or chloroform and under acidic conditions.

US 5,155,018 discloses a RNA extraction technique for allowing RNA to selectively bind to a solid phase from a biological sample in which DNA and RNA coexist using an acidified chaotropic agent.

### SUMMARY OF THE INVENTION

The purpose of this invention is to provide a method to extract selectively RNA with high purity from biological materials containing RNA in a safe, rapid, and simple procedure and a method to analyze it.

The present inventors discovered that RNA binds to silica with very high selectivity in the presence of a predetermined concentration of a chaotropic agent and a predetermined concentration of diethylene glycol dimethyl ether or ethyl lactate, and have succeeded in establishing a method for selective extraction of RNA and a method for analyzing RNA of the present invention.

The present invention includes the steps of mixing a biological material containing RNA with a predetermined concentration of a chaotropic agent and a predetermined concentration of diethylene glycol dimethyl ether or ethyl lactate, allowing the mixed solution to contact a nucleic acid-binding solid phase including silica, washing the nucleic-acid binding solid-phase to which RNA is bound, and eluting RNA from the nucleic-acid binding solid-phase having the bound RNA. Furthermore, the present invention relates to analyzing the obtained RNA by reverse transcriptase polymerase chain reaction.

According to the present invention, RNA can be extracted with very high purity. Since the extracted product hardly contains DNA, the RT-PCR method for analysis of RNA that is otherwise sensitive to DNA and the like can be carried out without any procedure of DNA removal that has a possibility to impair RNA. Therefore, RNA analysis of a biological sample can be accomplished with high accuracy.

### BRIEF DESCRIPTION OF THE DRAWINGS

Fig. 1 represents a nucleic acid-capture chip used in a first example and a second example;
Fig. 2 is an electrophoretogram of nucleic acid extracts in the first example;
Fig. 3 is an electrophoretogram of nucleic acid extracts in the second example;
Fig. 4 is an electrophoretogram of nucleic acid extracts in a comparative example; and
Fig. 5 is an electrophoretogram of RT-PCR products.

### DETAILED DESCRIPTION OF THE PREFERRED EMBODIMENTS

The above and other novel features and effects of the present invention will hereinafter be explained with reference to the accompanying drawings. It should be noted that these drawings are merely used for explanations and do not limit the scope of right of the present invention as defined in the claims.

Biological materials containing RNA that become a subject of concern may include biological samples such as whole blood, serum, sputum, urine, tissues from a living body, cultured cells, and cultured microorganisms and materials containing crude RNA.

Solubilization of biological materials is carried out by a physical method that uses a mortar, ultrasound, microwave, homogenizer, or the like, a chemical method that uses a surface active agent, protein denaturant, or the like, or a biochemical method utilizing a proteinase, and by a method being a combination of these methods.

Preferred examples of chaotropic agents are sodium iodide, potassium iodide, sodium thiocyanate, guanidine thiocyanate, and guanidine hydrochloride.

As an organic solvent diethylene glycol dimethyl ether or ethyl lactate are used.

The selective RNA extraction method of the present invention is based on the effect of the selective binding of RNA to silica, and this effect can be obtained in the presence of a predetermined concentration of a chaotropic agent and a predetermined concentration of diethylene glycol dimethylether or ethyl lactate.

When guanidine thiocyanate is used as the chaotropic agent and diethylene glycol dimethyl ether is used as the organic solvent, RNA with high purity is obtained in good yield at a guanidine thiocyanate concentration ranging from 1.0 to 4.0 mol/l and a diethylene glycol dimethyl ether concentration ranging from 10 to 30% in the final mixed solution. In particular, RNA with very high purity is obtained in high yield at a guanidine thiocyanate concentration ranging from 1.5 to 2.0 mol/l and a diethylene glycol dimethyl ether concentration ranging from 15 to 25% in the final mixed solution.

When guanidine thiocyanate is used as the chaotropic agent and ethyl lactate is used as the organic solvent, RNA with high purity is obtained in good yield at a guanidine thiocyanate concentration ranging from 1.0 to 4.0 mol/l and an ethyl lactate concentration ranging from 20 to 40% in the final mixed solution. In particular, RNA with very high purity is obtained in high yield at a guanidine thiocyanate concentration ranging from 1.5 to 2.5 mol/l and an ethyl lactate concentration ranging from 25 to 35% in the final mixed solution.

The nucleic acid-binding solid phase include glass particles, silica particles, glass fiber filter paper, silica wool, or their crushed materials, and materials containing silicon dioxide such as diatomaceous earth.

The contact of nucleic acid-binding solid phase with the mixed solution is carried out by a method of stirring and mixing the solid phase and the mixed solution in a vessel or a method of passing the mixed solution through a column with the immobilized solid phase. After allowing the nucleic acid-binding solid phase and the mixed solution to contact each other, the solid phase is separated from the mixed solution.

Washing of the nucleic-acid binding solid phase with the bound nucleic acids is performed, for example, by allowing the solid phase to contact a washing solution, followed by separating the solid phase from the washing solution. It is preferred to use ethanol at a concentration of at least 75% for the washing solution so that the nucleic acids bound to the solid phase may not be eluted out and non-specifically bound substances may be removed efficiently.

Elution of nucleic acids from the nucleic acid-binding phase is carried out by means of allowing the solid phase to contact an elution solution and eluting the nucleic acids bound to the solid phase into the elution solution, followed by separating the eluate from the solid phase. The elution solution to be used is water, a low salt buffer, or the like that has been treated for removal of RNase or inactivation of RNase activity. When the elution is performed under warming, the elution efficiency is improved.

The eluate containing eluted nucleic acids may be immediately used for RT-PCR.

### EXAMPLES

### First Example

In the present example, RNA extraction from cultured cells was carried out using guanidine thiocyanate as a chaotropic agent and diethylene glycol dimethyl ether as an organic solvent.

### Extraction of RNA

In a first step, 600 µl of a cell lysis solution (4 mol/l guanidine thiocyanate, 10 mmol/l MES-KOH, pH 6.5) was added to pellets of cultured mouse myeloma cells (ca. 10⁶ cells)(Sp/O-Ag 14; product of Dainippon Pharmaceutical Co., Ltd.), and the cells were disrupted by a homogenizer (Handy Micro Homogenizer; manufactured by Microtec Co., Ltd.), thereby releasing intracellular nucleic acids.

In a second step, 600 µl of each aqueous solution of diethylene glycol dimethyl ether (20, 40, 60, 80, and 100% by volume) was added, as an organic solvent, to the cell lysate after the first step. At this time, the concentrations of guanidine thiocyanate became 2 mol/l, and those of diethylene glycol dimethyl ether became 10, 20, 30, 40, and 50% by volume, respectively, in the mixed solution.

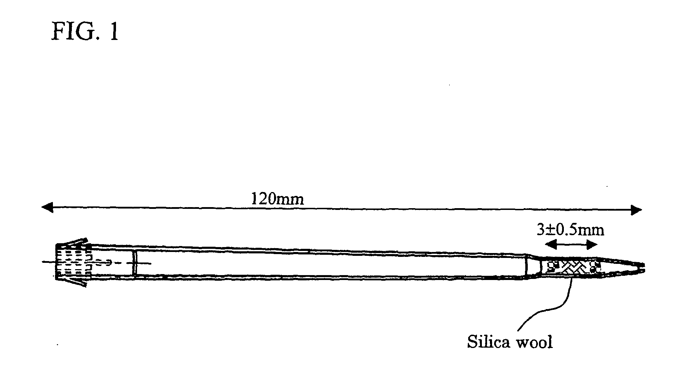

In a third step, a syringe (25 ml syringe; product of Terumo Corporation) was attached to a nucleic acid-capture chip made of polypropylene of which tip was packed with 5 mg of silica wool (B grade; Toshiba Chemical Corporation) as the nucleic acid-binding solid phase as shown in Fig. 1, and the solution after the second step was aspirated and dispensed, thereby allowing the solid phase to contact nucleic acids for separation.

In a fourth step, 1,200 µl of a washing solution (aqueous solution of 80% by volume ethanol) was aspirated and dispensed of the nucleic acid-capture chip, thereby allowing the solid phase to contact the washing solution, and thus, substances bound non-specifically to the solid phase were separated and removed.

In a fifth step, 100 µl of an elution solution (DEPC-treated water) was aspirated and dispensed of the nucleic acid-capture chip, thereby allowing the solid phase to contact the elution solution and be separated finally from the latter, and thus, an eluate containing purified nucleic acids was obtained.

### Evaluation of extracted RNA

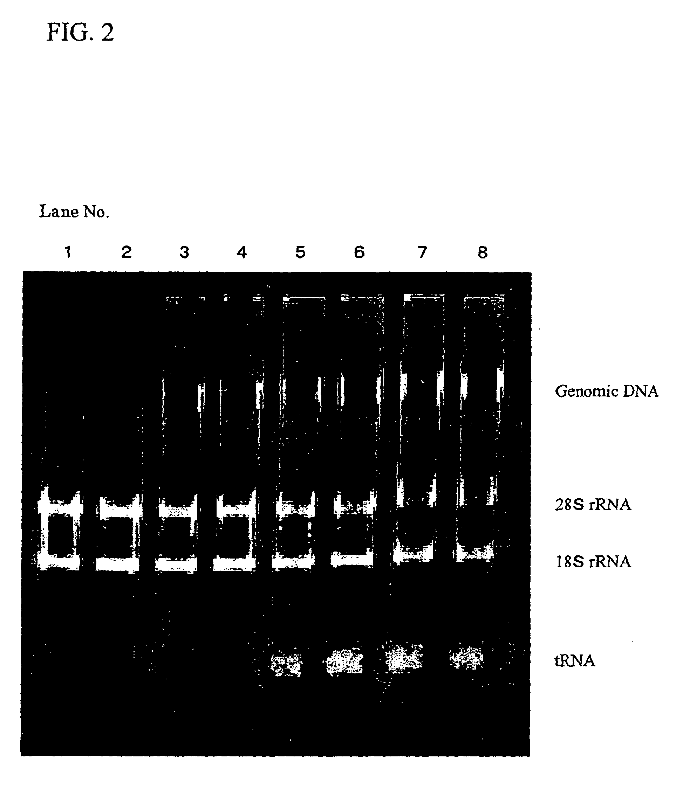

Fig. 2 shows the results of electrophoresis carried out for portions of the eluates on 1.25% agarose gel (Reliant RNA Gel System; product of FMC BioProducts) and its subsequent visualization by staining with ethidium bromide and taking a photograph under UV irradiation with a transilluminator. Lanes 1 and 2 represent nucleic acids extracted by the use of the aqueous solution of 40% by volume diethylene glycol dimethyl ether; lanes 3 and 4 represent nucleic acids extracted by the use of the aqueous solution of 60% by volume diethylene glycol dimethyl ether; lanes 5 and 6 represent nucleic acids extracted by the use of the aqueous solution of 80% by volume diethylene glycol dimethyl ether; and lanes 7 and 8 represent nucleic acids extracted by the use of the aqueous solution of 100% by volume diethylene glycol dimethyl ether.

Nucleic acids are separated by the electrophoresis according to their molecular weights. From the top of the electrophoretogram, bands corresponding to genomic DNA, 28S rRNA, 18S rRNA, and tRNA are shown, respectively. It is apparent from Fig. 2 that genomic DNA was hardly recognized and RNA with very high purity was obtained in high yield when the aqueous solution of 40% by volume diethylene glycol dimethyl ether was used. On the other hand, when the aqueous solutions of 60 to 100% by volume diethylene glycol dimethyl ether were used, it is apparent that the nucleic acid extracts contained large amounts of genomic DNA. In addition, when the aqueous solution of 20% by volume diethylene glycol dimethyl ether was used, nucleic acids were hardly obtained by the extraction.

### Second Example

In the present example, RNA extraction from cultured cells was carried out using guanidine thiocyanate as the chaotropic agent and ethyl lactate as the organic solvent.

### Extraction of RNA

The extraction of RNA of the present embodiment was conducted in the same manner as in the first embodiment except for the second step. The second step is described below.

In the second step, 600 µl of each aqueous solution of ethyl lactate (20, 40, 60, 80, and 100% by volume) was added, as the organic solvent, to the cell lysate after the first step. At this time, the concentrations of guanidine thiocyanate became 2 mol/l, and those of ethyl lactate became 10, 20, 30, 40, and 50% by volume, respectively, in the mixed solution.

### Evaluation of extracted RNA

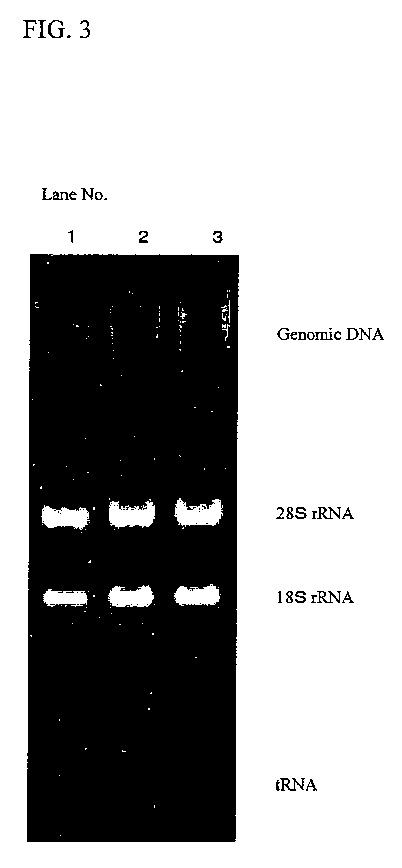

Fig. 3 shows the results of electrophoresis carried out in the same manner as in the first embodiment. Lane 1 represents nucleic acids extracted by the use of the aqueous solution of 60% by volume ethyl lactate; lane 2 represents nucleic acids extracted by the use of the aqueous solution of 80% by volume ethyl lactate; and lane 3 represents nucleic acids extracted by the use of the aqueous solution of 100% by volume ethyl lactate.

It is shown here that genomic DNA was hardly recognized and that RNA with very high purity was obtained in high yield when the aqueous solution of 60% by volume ethyl lactate was used. On the other hand, when the aqueous solutions of 80 and 100% by volume ethyl lactate were used, it is apparent that the nucleic acid extracts contained large amounts of genomic DNA. In addition, when the aqueous solutions of 20 and 40% by volume ethyl lactate were used, nucleic acids were hardly obtained by the extraction.

### Comparative Example

In the present embodiment, RNA extraction from cultured cells was carried out with the RNA extraction kit (RNeasy Mini Kit; product of Qiagen Inc.) that uses guanidine thiocyanate as the chaotropic agent and ethanol as the organic solvent. This method is based on the method disclosed in Patent document 1 described above.

### Extraction of RNA

Extraction of RNA from pellets of cultured mouse myeloma cells (ca. 10⁶ cells) that were the same as those used in the first embodiment was conducted using the RNeasy Mini Kit obtained from Qiagen according to the protocol attached to the kit.

### Evaluation of extracted RNA

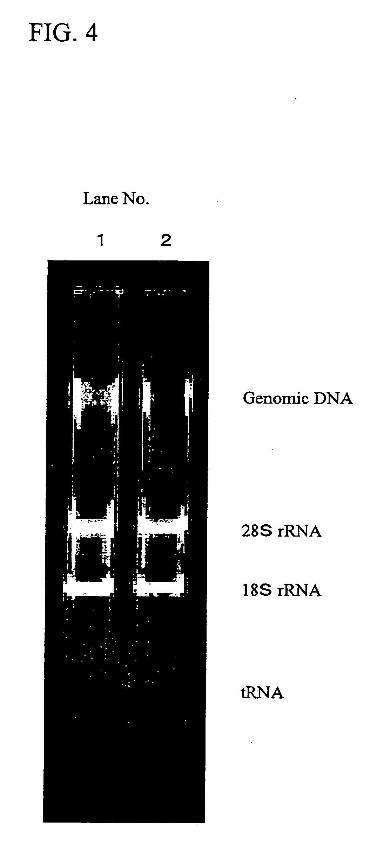

Fig. 4 shows the results of electrophoresis carried out in the same manner as in the first embodiment. These results indicate that the nucleic acid extracts contained genomic DNA when the RNeasy Mini Kit was used.

### RT-PCR with nucleic acid extracts

RT-PCR was carried out using the nucleic acid extracts obtained in the first embodiment and those obtained by the method of the comparative example.

Nucleic acid solutions each containing 2.5 µg of total RNA were prepared, respectively, from the nucleic acids extracted according to the methods of the first embodiment and the comparative example without performing a DNA removal procedure. To each of these nucleic acid solutions was added a reverse transcriptase (Superscript II; product of Invitrogen Corporation) and reagents for reverse transcription containing an oligo (dT) primer. The final volume was adjusted to 20 µl, and incubated for 50 min at 42 degrees C, thereby allowing cDNA to be synthesized by the reverse transcription reaction with mRNA as the template.

To 2 µl and 0.2 µl of the solution after the reverse transcription reaction were then added PCR primers targeted to a region of mouse β-actin gene not containing intron (Mouse β-actin RT-PCR Primer Set; product of Toyobo Co., Ltd.), a thermostable DNA polymerase (AmpliTaq Gold DNA polymerase; product of Applied Biosystems), and reagents for PCR. The final volume was adjusted to 50 µl, and a cycle of 94 degrees C for 15 sec, 55 degrees C for 30 sec, and 72 degrees C for 1 min was repeated 30 times using a thermal cycler (GeneAmp PCR System 9600; manufactured by PerkinElmer, Inc.).

PCR was carried out using 2 µl and 0.2 µl of the non-reacted solution without subjecting to the reverse transcription reaction as negative controls and DNA originating from mouse β-actin gene that was supplied with the PCR primers (Mouse β-actin RT-PCR Primer Set; product of Toyobo Co., Ltd.) as a positive control.

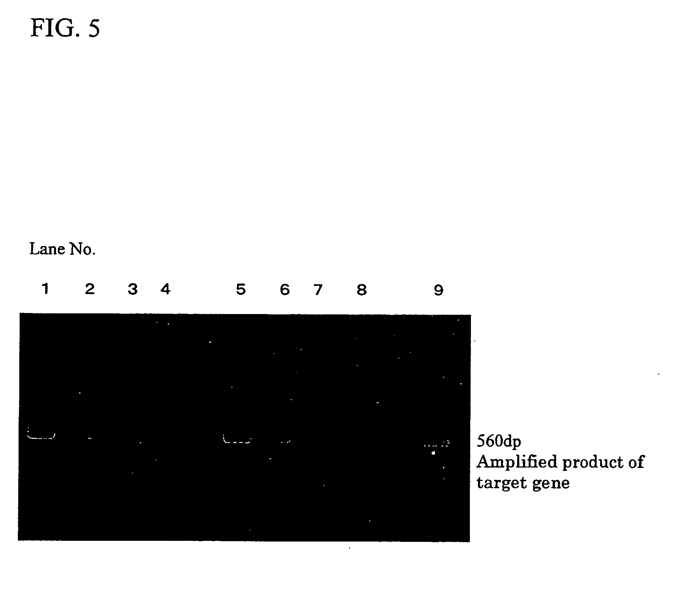

After PCR reaction, the solution was subjected to electrophoresis on 3% agarose gel (Nusieve 3:1 Agarose; product of FMC BioProducts). Fig. 5 shows the results of the electrophoretogram that was visualized by taking a photograph under UV irradiation with a transilluminator after staining with ethidium bromide.

In Fig. 5, lane 1 represents an amplified product that was obtained by the reverse transcription reaction using the nucleic acids extracted according to the method described in the first embodiment, followed by PCR amplification of 2 µl of the solution after the reverse transcription reaction. Lane 2 represents an amplified product that was obtained by the reverse transcription reaction using the nucleic acids extracted according to the method described in the first embodiment, followed by PCR amplification of 0.2 µl of the solution after the reverse transcription reaction. Lane 3 represents an amplified product that was obtained by direct PCR amplification of 2 µl of the unreacted solution in which the nucleic acids extracted according to the method described in the first embodiment were not subjected to the reverse transcription reaction. Lane 4 represents an amplified product that was obtained by direct PCR amplification of 0.2 µl of the unreacted solution in which the nucleic acids extracted according to the method described in the first embodiment were not subjected to the reverse transcription reaction.

Lane 5 represents an amplified product that was obtained by the reverse transcription reaction using the nucleic acids extracted according to the method described in the comparative example, followed by PCR amplification of 2 µl of the solution after the reverse transcription reaction. Lane 6 represents an amplified product that was obtained by the reverse transcription reaction using the nucleic acids extracted according to the method described in the comparative example, followed by PCR amplification of 0.2 µl of the solution after the reverse transcription reaction. Lane 7 represents an amplified product that was obtained by direct PCR amplification of 2 µl of the unreacted solution in which the nucleic acids extracted according to the method described in the comparative example were not subjected to the reverse transcription reaction. Lane 8 represents an amplified product that was obtained by direct PCR amplification of 0.2 µl of the unreacted solution in which the nucleic acids extracted according to the method described in the comparative example were not subjected to the reverse transcription reaction. Lane 9 represents an amplified product that was obtained by PCR amplification using DNA originating from mouse β-actin gene as the positive control.

From these results, the amplified product of 540 bp originating from mouse β-actin gene was confirmed in lanes 1, 2, 5, 6, 7, and 9. The amplified product was not confirmed when the nucleic acids extracted according to the method of the first embodiment were not subjected to the reverse transcription reaction (Lanes 3 and 4). This suggests that the amplified product (Lanes 1 and 2) after the reverse transcription reaction was derived from mRNA and that RT-PCR can be carried out without removing genomic DNA from the nucleic acid extracts.

On the other hand, the nucleic acids extracted according to the method described in the comparative example gave rise to an amplified product when 2 µl of the unreacted solution without being subjected to the reverse transcription reaction was used (Lane 7). This product is an amplification product derived from the genomic DNA that was contained in the nucleic acid extracts. Accordingly, an amplified product that was obtained by PCR using 2 µl of the solution after the reverse transcription reaction (Lane 5) is likely to be a mixture of amplification products derived from mRNA and genomic DNA, which suggests that RT-PCR does not function properly in this case. When RT-PCR is carried out with the nucleic acids extracted according to the method of the comparative example, it is therefore necessary to remove genomic DNA in advance from the nucleic acid extracts.

## Claims

1. A method for extracting RNA comprising the steps of:
mixing an RNA-containing biological material with diethylene glycol dimethyl ether, a chaotropic agent, and a nucleic acid-binding solid phase including silica to allow RNA to bind selectively to the solid phase;
separating the solid phase bound to the RNA from a liquid phase;
washing the solid phase; and
eluting the RNA from the solid phase, wherein
the concentration of the chaotropic agent in the mixed solution comprising the RNA-containing biological material, the diethylene glycol dimethyl ether, and the chaotropic agent ranges from 1.0 to 4.0 mol/l, and wherein
the concentration of the diethylene glycol dimethyl ether in the mixed solution ranges from 10 to 30 %.

2. A method for extracting RNA comprising the steps of:
mixing an RNA-containing biological material with ethyl lactate, a chaotropic agent, and a nucleic acid-binding solid phase including silica to allow RNA to bind selectively to the solid phase;
separating the solid phase bound to the RNA from a liquid phase;
washing the solid phase; and
eluting the RNA from the solid phase, wherein
the concentration of the chaotropic agent in the mixed solution comprising the RNA-containing biological material, the ethyl lactate, and the chaotropic agent ranges from 1.0 to 4.0 mol/l, and wherein
the concentration of the ethyl lactate in the mixed solution ranges from 20 to 40 %.

3. The method for extracting RNA according to claim 1 or 2, wherein the chaotropic agent is at least one selected from the group consisting of sodium iodide, potassium iodide, sodium thiocyanate, guanidine thiocyanate, and guanidine hydrochloride.

4. The method for extracting RNA according to claim 1, wherein a predetermined concentration of guanidine thiocyanate ranges from 1.0 to 4.0 mol/l with respect to the concentration in the mixed solution comprising the RNA-containing biological material, the diethylene glycol dimethyl ether, and the chaotropic agent, and the concentration of the diethylene glycol dimethyl ether in the mixed solution ranges from 10 to 30 %.

5. The method for extracting RNA according to claim 1, wherein a predetermined concentration of guanidine thiocyanate ranges from 1.5 to 2.0 mol/l with respect to the concentration in the mixed solution comprising the RNA-containing biological material, the diethylene glycol dimethyl ether, and the chaotropic agent, and the concentration of the diethylene glycol dimethyl ether in the mixed solution ranges from 15 to 25 %.

6. The method for extracting RNA according to claim 2, wherein a predetermined concentration of guanidine thiocyanate ranges from 1.0 to 4.0 mol/l with respect to the concentration in the mixed solution comprising the RNA-containing biological material, the ethyl lactate, and the chaotropic agent, and the concentration of the ethyl lactate in the mixed solution ranges from 20 to 40 %.

7. The method for extracting RNA according to claim 2, wherein a predetermined concentration of guanidine thiocyanate ranges from 1.5 to 2.5 mol/l with respect to the concentration in the mixed solution comprising the RNA-containing biological material, the ethyl lactate, and the chaotropic agent, and the concentration of the ethyl lactate in the mixed solution ranges from 25 to 35 %.

8. The method for extracting RNA according to any one of claims 1 to 7, wherein the nucleic acid-binding solid phase including silicon oxide is a glass particle, a silica particle, glass fiber filter paper, silica wool, a crushed material thereof, or diatomaceous earth.

9. The method for extracting RNA according to any one of claims 1 to 8, wherein the RNA-containing biological material is whole blood, serum, sputum, urine, biological tissue, cultured cells, or cultured bacteria.

10. The method for extracting RNA according to any one of claims 1 to 9, wherein cleaning fluid is ethanol.

11. The method for extracting RNA according to any one of claims 1 to 10, wherein eluent is water or a low salt buffer solution treated to have ribonuclease removed or ribonuclease inactivated.

12. A method for analyzing a biological material comprising the steps of:
mixing an RNA-containing biological material with diethylene glycol dimethyl ether, a chaotropic agent, and a nucleic acid-binding solid phase including silica to allow RNA to bind selectively to the solid phase;
separating the solid phase bound to the RNA from a liquid phase;
washing the solid phase;
eluting the RNA from the solid phase; and
amplifying the gained RNA using reverse transcription polymerase chain reaction (RT-PCR), wherein
the concentration of the chaotropic agent in the mixed solution comprising the RNA-containing biological material, the diethylene glycol dimethyl ether, and the chaotropic agent ranges from 1.0 to 4.0 mol/l, and wherein
the concentration of the diethylene glycol dimethyl ether in the mixed solution ranges from 10 to 3 0 %.

13. A method for analyzing a biological material comprising the steps of:
mixing an RNA-containing biological material with ethyl lactate, a chaotropic agent, and a nucleic acid-binding solid phase including silica to allow RNA to bind selectively to the solid phase;
separating the solid phase bound to the RNA from a liquid phase;
washing the solid phase;
eluting the RNA from the solid phase; and
amplifying the gained RNA using reverse transcription polymerase chain reaction (RT-PCR), wherein
the concentration of the chaotropic agent in the mixed solution comprising the RNA-containing biological material, the ethyl lactate, and the chaotropic agent ranges from 1.0 to 4.0 mol/l, and wherein
the concentration of the ethyl lactate in the mixed solution ranges from 20 to 40%.

14. The method for analyzing a biological material according to claim 12 or 13, wherein the chaotropic agent is at least one selected from the group consisting of sodium iodide, potassium iodide, sodium thiocyanate, guanidine thiocyanate, and guanidine hydrochloride.

15. The method for analyzing a biological material according to claim 12, wherein a predetermined concentration of guanidine thiocyanate ranges from 1.0 to 4.0 mol/l with respect to the concentration in the mixed solution comprising the RNA-containing biological material, the diethylene glycol dimethyl ether, and the chaotropic agent, and the concentration of the diethylene glycol dimethyl ether in the mixed solution ranges from 10 to 30 %.

16. The method for analyzing a biological material according to claim 12, wherein a predetermined concentration of guanidine thiocyanate ranges from 1.5 to 2.0 mol/l with respect to the concentration in the mixed solution comprising the RNA-containing biological material, the diethylene glycol dimethyl ether, and the chaotropic agent, and the concentration of the diethylene glycol dimethyl ether in the mixed solution ranges from 15 to 25 %.

17. The method for analyzing a biological material according to claim 13, wherein a predetermined concentration of guanidine thiocyanate ranges from 1.0 to 4.0 mol/l with respect to the concentration in the mixed solution comprising the RNA-containing biological material, the ethyl lactate, and the chaotropic agent, and the concentration of the ethyl lactate in the mixed solution ranges from 20 to 40 %.

18. The method for analyzing a biological material according to claim 13, wherein a predetermined concentration of guanidine thiocyanate ranges from 1.5 to 2.5 mol/l with respect to the concentration in the mixed solution comprising the RNA-containing biological material, the ethyl lactate, and the chaotropic agent, and the concentration of the ethyl lactate in the mixed solution ranges from 25 to 35 %.

19. The method for analyzing a biological material according to any one of claims 12 to 18, wherein the nucleic acid-binding solid phase including silicon oxide is a glass particle, a silica particle, glass fiber filter paper, silica wool, a crushed material thereof, or diatomaceous earth.

20. The method for analyzing a biological material according to any one of claims 12 to 19, wherein the RNA-containing biological material is whole blood, blood serum, sputum, urine, biological tissue, cultured cells, or cultured bacteria.

21. The method for analyzing a biological material according to any one of claims 12 to 20, wherein cleaning fluid is ethanol.

22. The method for analyzing a biological material according to any one of claims 12 to 21, wherein eluent is water or a low salt concentration buffer solution treated to have ribonuclease removed or ribonuclease inactivated.

23. The method for extracting RNA according to any one of claims 1 to 11, wherein silica is silicon dioxide.

24. The method for analyzing a biological material according to any one of claims 12 to 22, wherein silica is silicon: dioxide.

## Patentansprüche

1. Verfahren zum Extrahieren von RNA, welches die folgenden Stufen umfasst:
Mischen eines RNA enthaltenden biologischen Materials mit Diethylenglycoldiethylether, einem chaotropen Mittel und einer Nucleinsäure bindenden festen Phase, einschließlich Silicamaterial, um die selektive Bindung von RNA an die feste Phase zuzulassen;
Abtrennen der an die RNA gebundenen festen Phase von der flüssigen Phase;
Waschen der festen Phase; und
Eluieren der RNA von der festen Phase, wobei
die Konzentration des chaotropen Mittels in der Mischlösung, die das RNA enthaltende biologische Material, den Diethylenglycoldimethylether und das chaotrope Mittel enthält, im Bereich von 1,0 bis 4,0 mol/l ist, und wobei
die Konzentration des Diethylenglycoldimethylethers in der Mischlösung im Bereich von 10 bis 30 % ist.

2. Verfahren zum Extrahieren von RNA, welches die folgenden Stufen umfasst:
Mischen eines RNA enthaltenden biologischen Materials mit Ethyllactat, einem chaotropen Mittel und einer Nucleinsäure bindenden festen Phase, einschließlich Silicamaterial, um die selektive Bindung von RNA an die feste Phase zuzulassen;
Abtrennen der an die RNA gebundenen festen Phase von der flüssigen Phase;
Waschen der festen Phase; und
Eluieren der RNA von der festen Phase, wobei
die Konzentration des chaotropen Mittels in der Mischlösung, die das RNA enthaltende biologische Material , das Ethyllactat und das chaotrope Mittel enthält, im Bereich von 1,0 bis 4,0 mol/l ist, und wobei
die Konzentration des Ethyllactats in der Mischlösung im Bereich von 20 bis 40 % ist.

3. Verfahren zum Extrahieren von RNA nach Anspruch 1 oder 2, wobei das chaotrope Mittel mindestens eines ist, das aus der Gruppe ausgewählt ist, die aus Natriumiodid, Kaliumiodid, Natriumthiocyanat, Guanidinthiocyanat und Guanidinhydrochlorid besteht.

4. Verfahren zum Extrahieren von RNA nach Anspruch 1, wobei eine vorbestimmte Konzentration von Guanidinthiocyanat im Bereich von 1,0 bis 4,0 mol/l, bezogen auf die Konzentration in der Mischlösung, die das RNA enthaltende biologische Material, den Diethylenglycoldimethylether und das chaotrope Mittel enthält, liegt und die Konzentration des Diethylenglycoldimethylethers in der Mischlösung im Bereich von 10 bis 30 % ist.

5. Verfahren zum Extrahieren von RNA nach Anspruch 1, wobei eine vorbestimmte Konzentration von Guanidinthiocyanat im Bereich von 1,5 bis 2,0 mol/l, bezogen auf die Konzentration in der Mischlösung, die das RNA enthaltende biologische Material, dem Diethylenglycoldimethylether und das chaotrope Mittel enthält, liegt und die Konzentration des Diethylenglycoldimethylethers in der Mischlösung im Bereich von 15 bis 25 % liegt.

6. Verfahren zum Extrahieren von RNA nach Anspruch 2, wobei eine vorbestimmte Konzentration von Guanidinthiocyanat im Bereich von 1,0 bis 4,0 mol/l, bezogen auf die Konzentration in der Mischlösung, die das RNA enthaltende biologische Material, das Ethyllactat und das chaotrope Mittel enthält, liegt und die Konzentration des Ethyllactats in der Mischlösung im Bereich von 20 bis 40 % liegt.

7. Verfahren zum Extrahieren von RNA nach Anspruch 2, wobei eine vorbestimmte Konzentration von Guanidinthiocyanat im Bereich von 1,5 bis 2,5 mol/l, bezogen auf die Konzentration in der Mischlösung, die das RNA enthaltende biologische Material, das Ethyllactat und das chaotrope Mittel enthält, liegt und die Konzentration des Ethyllactats in der Mischlösung im Bereich von 25 bis 35 % liegt.

8. Verfahren zum Extrahieren von RNA nach einem der Ansprüche 1 bis 7, wobei die Nucleinsäure bindende feste Phase, die Siliciumoxid enthält, ein Glasteilchen, Kieselsäureteilchen, Glasfaserfilterpapier, Quarzwolle, ein zerstoßenes Produkt davon oder Diatomeenerde ist.

9. Verfahren zum Extrahieren von RNA nach einem der Ansprüche 1 bis 8, wobei das RNA enthaltende biologische Material Gesamtblut, Serum, Auswurf, Urin, biologisches Gewebe, kultivierte Zellen oder kultivierte Bakterien ist.

10. Verfahren zum Extrahieren von RNA nach einem der Ansprüche 1 bis 9, wobei das Reinigungsfluid Ethanol ist.

11. Verfahren zum Extrahieren von RNA nach einem der Ansprüche 1 bis 10, wobei das Elutionsmittel Wasser oder eine Niedrigsalzpufferlösung ist, wobei das Elutionsmittel behandelt worden ist, um Ribonuclease zu entfernen oder zu inaktivieren.

12. Verfahren zum Analysieren eines biologischen Materials, welches die folgenden Stufen umfasst:
Mischen eines RNA enthaltenden biologischen Materials mit Diethylenglycoldiethylether, einem chaotropen Mittel und einer Nucleinsäure bindenden festen Phase, einschließlich Silicamaterial, um die selektive Bindung von RNA an die feste Phase zuzulassen;
Abtrennen der an die RNA gebundenen festen Phase von der flüssigen Phase;
Waschen der festen Phase; und
Eluieren der RNA von der festen Phase; und
Amplifizieren der erhaltenen RNA unter Einsatz einer Polymerasekettenreaktion mit einer reversen Transkriptase (RT-PCR), wobei
die Konzentration des chaotropen Mittels in der Mischlösung, die das RNA enthaltende biologische Material, den Diethylenglycoldimethylether und das chaotrope Mittel enthält, im Bereich von 1,0 bis 4,0 mol/l ist, und wobei
die Konzentration des Diethylenglycoldimethylethers in der Mischlösung im Bereich von 10 bis 30 % ist.

13. Verfahren zum Analysieren eines biologischen Materials, welches die folgenden Stufen umfasst:
Mischen eines RNA enthaltenden biologischen Materials mit Diethylenglycoldiethylether, einem chaotropen Mittel und einer Nucleinsäure bindenden festen Phase, einschließlich Silicamaterial, um die selektive Bindung von RNA an die feste Phase zuzulassen;
Abtrennen der an die RNA gebundenen festen Phase von der flüssigen Phase;
Waschen der festen Phase; und
Eluieren der RNA von der festen Phase; und
Amplifizieren der erhaltenen RNA unter Einsatz einer Polymerasekettenreaktion mit einer reversen Transkriptase (RT-PCR), wobei
die Konzentration des chaotropen Mittels in der Mischlösung, die das RNA enthaltende biologische Material, den Diethylenglycoldimethylether und das chaotrope Mittel enthält, im Bereich von 1,0 bis 4,0 mol/l ist, und wobei
die Konzentration des Diethylenglycoldimethylethers in der Mischlösung im Bereich von 20 bis 40 % ist.

14. Verfahren zum Analysieren eines biologischen Materials nach Anspruch 12 oder 13, wobei das chaotrope Mittel mindestens eines ist, das aus der Gruppe ausgewählt ist, die aus Natriumiodid, Kaliumiodid, Natriumthiocyanat, Guanidinthiocyanat und Guanidinhydrochlorid besteht.

15. Verfahren zum Analysieren eines biologischen Materials nach Anspruch 12, wobei eine vorbestimmte Konzentration von Guanidinthiocyanat im Bereich von 1,0 bis 4,0 mol/l, bezogen auf die Konzentration in der Mischlösung, die das RNA enthaltende biologische Material, den Diethylenglycoldimethylether und das chaotrope Mittel enthält, liegt und die Konzentration des Diethylenglycoldimethylethers in der Mischlösung im Bereich von 10 bis 30 % ist.

16. Verfahren zum Analysieren eines biologischen Materials nach Anspruch 12, wobei eine vorbestimmte Konzentration von Guanidinthiocyanat im Bereich von 1,5 bis 2,0 mol/l, bezogen auf die Konzentration in der Mischlösung, die das RNA enthaltende biologische Material, den Diethylenglycoldimethylether und das chaotrope Mittel enthält, liegt und die Konzentration des Diethylenglycoldimethylethers in der Mischlösung im Bereich von 15 bis 25 % ist.

17. Verfahren zum Analysieren eines biologischen Materials nach Anspruch 12, wobei eine vorbestimmte Konzentration von Guanidinthiocyanat im Bereich von 1,0 bis 4,0 mol/l, bezogen auf die Konzentration in der Mischlösung, die das RNA enthaltende biologische Material, das Ethyllactat und das chaotrope Mittel enthält, liegt und die Konzentration des Ethyllactats in der Mischlösung im Bereich von 20 bis 40 % liegt.

18. Verfahren zum Analysieren eines biologischen Materials nach Anspruch 12, wobei eine vorbestimmte Konzentration von Guanidinthiocyanat im Bereich von 1,5 bis 2,5 mol/l, bezogen auf die konzentration in der Mischlösung, die das RNA enthaltende biologische Material, das Ethyllactat und das chaotrope Mittel enthält, liegt und die Konzentration des Ethyllactats in der Mischlösung im Bereich von 25 bis 35 % liegt.

19. Verfahren zum Analysieren eines biologischen Materials nach einem der Ansprüche 12 bis 18, wobei die Nucleinsäure bindende feste Phase, die Siliciumoxid enthält, ein Glasteilchen, Kieselsäureteilchen, Glasfaserfilterpapier, Quarzwolle, ein zerstoßenes Produkt davon oder Diatomeenerde ist.

20. Verfahren zum Analysieren eines biologischen Materials nach einem der Ansprüche 12 bis 19, wobei das RNA enthaltende biologische Material Gesamtblut, Serum, Auswurf, Urin, biologisches Gewebe, kultivierte Zellen oder kultivierte Bakterien ist.

21. Verfahren zum Analysieren eines biologischen Materials nach einem der Ansprüche 12 bis 20, wobei das Reinigungsfluid Ethanol ist.

22. Verfahren zum Analysieren eines biologischen Materials nach einem der Ansprüche 12 bis 21, wobei das Elutionsmittel Wasser oder eine Niedrigsalzpufferlösung ist, wobei das Elutionsmittel behandelt worden ist, um Ribonuclease zu entfernen oder zu inaktivieren.

23. Verfahren zum Extrahieren von RNA nach einem der Ansprüche 1 bis 11, wobei das Silicamaterial Siliciumdioxid ist.

24. Verfahren zum Analysieren eines biologischen Materials nach einem der Ansprüche 12 bis 22, wobei das Silicamaterial Siliciumdioxid ist.

## Revendications

1. Procédé d'extraction d'ARN comprenant les étapes consistant à :
mélanger un matériau biologique contenant de l'ARN avec de l'éther diméthylique de diéthylèneglycol, un agent chaotropique et une phase solide liant les acides nucléiques incluant de la silice, pour permettre à l'ARN de se lier sélectivement à la phase solide ;
séparer la phase solide liée à l'ARN de la phase liquide ;
laver la phase solide ; et
éluer l'ARN de la phase solide, dans lequel
la concentration de l'agent chaotropique dans la solution mixte comprenant le matériau biologique contenant de l'ARN, l'éther diméthylique de diéthylèneglycol et l'agent chaotropique varie de 1,0 mol/ à 4,0 mol/l, et dans lequel la concentration d'éther diméthylique de diéthylèneglycol dans la solution mixte varie de 10 à 30 %.

2. Procédé d'extraction d'ARN comprenant les étapes consistant à :
mélanger un matériau biologique contenant de l'ARN avec du lactate d'éthyle, un agent chaotropique et une phase solide liant les acides nucléiques incluant de la silice pour permettre à l'ARN de se lier sélectivement à la phase solide ;
séparer la phase solide liée à l'ARN de la phase liquide ;
laver la phase solide ; et
éluer l'ARN de la phase solide, dans lequel
la concentration de l'agent chaotropique dans la solution mixte comprenant le matériau biologique contenant de l'ARN, le lactate d'éthyle et l'agent chaotropique varie de 1,0 mol/ à 4,0 mol/l, et dans lequel
la concentration de lactate d'éthyle dans la solution mixte varie de 20 à 40 %.

3. Procédé d'extraction d'ARN selon la revendication 1 ou 2, dans lequel l'agent chaotropique est au moins un agent choisi dans l'ensemble consistant en iodure de sodium, iodure de potassium, thiocyanate de sodium, thiocyanate de guanidine et chlorhydrate de guanidine.

4. Procédé d'extraction d'ARN selon la revendication 1, dans lequel une concentration prédéterminée de thiocyanate de guanidine varie de 1,0 à 4,0 mol/l en termes de concentration dans la solution mixte comprenant le matériau biologique contenant de l'ARN, l'éther diméthylique de diéthylèneglycol et l'agent chaotropique, et la concentration d'éther diméthylique de diéthylèneglycol dans la solution mixte varie de 10 à 30 %.

5. Procédé d'extraction d'ARN selon la revendication 1, dans lequel une concentration prédéterminée de thiocyanate de guanidine varie de 1,5 à 2,0 mol/l en termes de concentration dans la solution mixte comprenant le matériau biologique contenant de l'ARN, l'éther diméthylique de diéthylèneglycol et l'agent chaotropique, et la concentration d'éther diméthylique de diéthylèneglycol dans la solution mixte varie de 15 à 25 %.

6. Procédé d'extraction d'ARN selon la revendication 2, dans lequel une concentration prédéterminée de thiocyanate de guanidine varie de 1,0 à 4,0 mol/l en termes de concentration dans la solution mixte comprenant le matériau biologique contenant de l'ARN, le lactate d'éthyle et l'agent chaotropique, et la concentration de lactate d'éthyle dans la solution mixte varie de 20 à 40 %.

7. Procédé d'extraction d'ARN selon la revendication 2, dans lequel une concentration prédéterminée de thiocyanate de guanidine varie de 1,5 à 2,5 mol/l en termes de concentration dans la solution mixte comprenant le matériau biologique contenant de l'ARN, le lactate d'éthyle et l'agent chaotropique, et la concentration de lactate d'éthyle dans la solution mixte varie de 25 à 35 %.

8. Procédé d'extraction d'ARN selon l'une quelconque des revendication 1 à 7, dans lequel la phase solide liant les acides nucléiques incluant de l'oxyde silicium est une particule de verre, une particule de silice, un papier filtre en fibres de verre, de la laine de silice, un matériau broyé fait à partir de ceux-ci ou une terre de diatomées.

9. Procédé d'extraction d'ARN selon l'une quelconque des revendication 1 à 8, dans lequel le matériau biologique contenant de l'ARN est du sang entier, du sérum, un crachat, de l'urine, un tissu biologique, des cellules cultivées ou des bactéries cultivées.

10. Procédé d'extraction d'ARN selon l'une quelconque des revendication 1 à 9, dans lequel le fluide de nettoyage est l'éthanol.

11. Procédé d'extraction d'ARN selon l'une quelconque des revendication 1 à 10, dans lequel l'éluant est de l'eau ou une solution tampon à faible teneur en sel traitée pour en éliminer les ribonucléases ou en inactiver les ribonucléases.

12. Procédé d'analyse d'un matériau biologique comprenant les étapes consistant à :
mélanger un matériau biologique contenant de l'ARN avec de l'éther diméthylique de diéthylèneglycol, un agent chaotropique et une phase solide liant les acides nucléiques incluant de la silice, pour permettre à l'ARN de se lier sélectivement à la phase solide ;
séparer la phase solide liée à l'ARN de la phase liquide;
laver la phase solide ;
éluer l'ARN de la phase solide ; et
amplifier l'ARN obtenu en utilisant une réaction en chaîne de polymérase avec transcription inverse (RT-PCR), dans lequel
la concentration de l'agent chaotropique dans la solution mixte comprenant le matériau biologique contenant de l'ARN, l'éther diméthylique de diéthylèneglycol et l'agent chaotropique varie de 1,0 mol/ à 4,0 mol/l, et dans lequel la concentration d'éther diméthylique de diéthylèneglycol dans la solution mixte varie de 10 à 30 %.

13. Procédé d'analyse d'un matériau biologique comprenant les étapes consistant à :
mélanger un matériau biologique contenant de l'ARN avec du lactate d'éthyle, un agent chaotropique et une phase solide liant les acides nucléiques incluant de la silice, pour permettre à l'ARN de se lier sélectivement à la phase solide ;
séparer la phase solide liée à l'ARN de la phase liquide ;
laver la phase solide ;
éluer l'ARN de la phase solide ; et
amplifier l'ARN obtenu en utilisant une réaction en chaîne de polymérase avec transcription inverse (RT-PCR), dans lequel
la concentration de l'agent chaotropique dans la solution mixte comprenant le matériau biologique contenant de l'ARN, le lactate d'éthyle et l'agent chaotropique varie de 1,0 mol/ à 4,0 mol/l, et dans lequel
la concentration de lactate d'éthyle dans la solution mixte varie de 10 à 30 %.

14. Procédé d'analyse d'un matériau biologique selon la revendication 12 ou 13, dans lequel l'agent chaotropique est au moins un agent choisi dans l'ensemble consistant en iodure de sodium, iodure de potassium, thiocyanate de sodium, thiocyanate de guanidine et chlorhydrate de guanidine.

15. Procédé d'analyse d'un matériau biologique selon la revendication 12, dans lequel une concentration prédéterminée de thiocyanate de guanidine varie de 1,0 à 4,0 mol/l en termes de concentration dans la solution mixte comprenant le matériau biologique contenant de l'ARN, l'éther diméthylique de diéthylèneglycol et l'agent chaotropique, et la concentration d'éther diméthylique de diéthylèneglycol dans la solution mixte varie de 10 à 30 %.

16. Procédé d'analyse d'un matériau biologique selon la revendication 12, dans lequel une concentration prédéterminée de thiocyanate de guanidine varie de 1,5 à 2,0 mol/l en termes de concentration dans la solution mixte comprenant le matériau biologique contenant de l'ARN, l'éther diméthylique de diéthylèneglycol et l'agent chaotropique, et la concentration d'éther diméthylique de diéthylèneglycol dans la solution mixte varie de 15 à 25 %.

17. Procédé d'analyse d'un matériau biologique selon la revendication 13, dans lequel une concentration prédéterminée de thiocyanate de guanidine varie de 1,0 à 4,0 mol/l en termes de concentration dans la solution mixte comprenant le matériau biologique contenant de l'ARN, le lactate d'éthyle et l'agent chaotropique, et la concentration de lactate d'éthyle dans la solution mixte varie de 20 à 40 %.

18. Procédé d'analyse d'un matériau biologique selon la revendication 13, dans lequel une concentration prédéterminée de thiocyanate de guanidine varie de 1,5 à 2,5 mol/l en termes de concentration dans la solution mixte comprenant le matériau biologique contenant de l'ARN, le lactate d'éthyle et l'agent chaotropique, et la concentration de lactate d'éthyle dans la solution mixte varie de 25 à 35 %.

19. Procédé d'analyse d'un matériau biologique selon l'une quelconque des revendication 12 à 18, dans lequel la phase solide liant les acides nucléiques incluant de l'oxyde silicium est une particule de verre, une particule de silice, un papier filtre en fibres de verre, de la laine de silice, un matériau broyé fait à partir de ceux-ci ou une terre de diatomées.

20. Procédé d'analyse d'un matériau biologique selon l'une quelconque des revendication 12 à 19, dans lequel le matériau biologique contenant de l'ARN est du sang entier, du sérum, un crachat, de l'urine, du tissu biologique, des cellules cultivées ou des bactéries cultivées.

21. Procédé d'analyse d'un matériau biologique selon l'une quelconque des revendication 12 à 20, dans lequel le fluide de nettoyage est l'éthanol.

22. Procédé d'analyse d'un matériau biologique selon l'une quelconque des revendication 12 à 21, dans lequel l'éluant est de l'eau ou une solution tampon à faible teneur en sel traitée pour en éliminer les ribonucléases ou en inactiver les ribonucléases.

23. Procédé d'extraction d'ARN selon l'une quelconque des revendications 1 à 11, dans lequel la silice est du dioxyde de silicium.

24. Procédé d'analyse d'un matériau biologique selon l'une quelconque des revendications 12 à 22, dans lequel la silice est du dioxyde de silicium.
